# EUROPEAN PATENT SPECIFICATION

(11) **EP 3 183 027 B1**
(45) Date of publication and mention of the grant of the patent: **28.01.2026**
(21) Application number: 15834444.0
(22) Date of filing: 21.08.2015
(51) Int. Cl.: A61B 5/0215, A61B 5/00

(54) **AUTOMATED ARTERIAL PRESSURE REGULATING DEVICE**
AUTOMATISIERTER ARTERIENDRUCKREGLER
DISPOSITIF DE RÉGULATION DE PRESSION ARTÉRIELLE AUTOMATISÉ

(30) Priority: 21.08.2014 US 201462039914 P
(43) Date of publication of application: 28.06.2017
(73) Proprietor: Harrity, M.D. William V., Pasadena, CA 91105 (US)
(72) Inventor: Harrity, M.D. William V., Pasadena, CA 91105 (US)
(74) Representative: Westphal, Mussgnug & Partner, Patentanwälte mbB
(86) International application number: PCT/US2015/046373
(87) International publication number: WO 2016/029155

(56) References cited:
- US-A1- 2011 133 935
- US-A1- 2011 190 692
- US-A1- 2013 116 663
- US-A1- 2013 226 138
- US-A1- 2013 261 472
- APPLIED PHYSIOLOGY: "Navigator. Guided Circulatory Management System, Training presentation v 2", vol. Navigator 0.92 v 1.0.0, 24 January 2007 (2007-01-24), pages 1 - 60, XP002644196, Retrieved from the Internet <URL:http://www.applied-physiology.com/media/Navigator__Presentation_Jan_24_2007_v2.pdf> [retrieved on 20110622]

## Description

### BACKGROUND

### Hypertension

Tight blood pressure control is critical during certain types of surgery and post-operatively in the ICU. Hypertensive emergencies in of themselves can cause severe bodily injury. In the eye severe hypertension can cause retinopathy and papilledema, in the brain hypertensive encephalopathy, cerebral infarction, and subarachnoid or intracranial hemorrhage, the cardiovascular system can experience myocardial ischemia and infarction, acute left ventricular dysfunction, acute pulmonary edema, and aortic dissection and the kidney can experience renal insufficiency and acute renal failure.

### Perioperative Hypertension

Hypertension in the perioperative period can have similar consequences as hypertensive emergencies. In addition perioperative hypertension can result in increased bleeding at the operative site. With respect to certain surgical procedures such as cardiac, vascular, or neurosurgical operations, hypertensive bleeding can be catastrophic.

### Hypotension

Hypotension can result in decreased blood flow to end organs causing ischemia and organ system failure such as ischemic stroke, myocardial infarction, acute renal failure, hepatic necrosis and if hypotension is prolonged it results in death.

### Perioperative Hypotension

Clinical studies have shown that tight blood pressure control in the operating room during cardiac surgery and perioperatively can reduce the risk of renal dysfunction, which has a major impact on quality of life. Many investigators have also found that intraoperative hypo or hypertensive events significantly influence the risk of perioperative cardiac morbidity. In the patient with coronary disease undergoing major surgery such as: emergency surgery, vascular surgery, prolonged thoracic or upper abdominal surgery or cardiac surgery, intraoperative hypotension is a major hemodynamic risk factor associated with significantly increased myocardial infarction rates. In patients with a previous myocardial infarction intraoperative hypotension has been found to result in a five-fold increase in the reinfarction rate. Although hypotension reduces myocardial demand and wall tension it also decreases coronary blood flow which can result in myocardial infarction. Perioperative hypotension can also result in ischemic stroke, renal failure and hepatic necrosis.

### Demand for Tight Blood Pressure Control

There is a demand for tight blood pressure control intra and postoperatively for certain surgical procedures as well as during the critical care of other medical situations where control of pressure within a certain range is paramount. Often surgeons give ICU nurses a blood pressure range that they would like their patients to remain in. This blood pressure range is often a compromise between the desire to control post-operative bleeding (as low a blood pressure as possible) and the maintenance of adequate end organ perfusion (a higher blood pressure usually around the patients baseline pressure). The ability to control pressure in a tight range is a difficult one at best. There are so many dynamic factors that come together to determine a patient's blood pressure at any one moment in time and those factors are constantly changing. It is not uncommon to witness a severe undershoot and/or an overshoot in the same patient with regards to requested blood pressure parameters.

### Intravenous Drugs Used to Control Blood Pressure

There are two basic types of intravenous drugs used to control blood pressure. Vasodilators are drugs that dilate vessels and decrease blood pressure and Vasoconstrictors that constrict blood vessels causing blood pressure to rise. Often it is not so simple and compounding factors such as the patient's current blood volume, sedation level, cardiac contractility and other vasoactive drugs can make pressure control difficult with these drugs. Each patient reacts differently to these drugs compounding the confusion so that no single dose is sufficient for any patient and each patient's needs are constantly changing; making the titration of these intravenous vasoactive drugs difficult for anesthesiologists and ICU personnel.

### Relevant Art

Different inventions have been patented that try to address this situation. European Patent number EP0408483 B1 prescribes the use of a single vasodilator with varied inputs such as the use of a BP cuff or invasive arterial pressure. This device however is broad in its description and involves the use of only a single drug to control only hypertension. US Patent number US8608683 - Device for infusion of at least two medicaments is a complex system that incorporates so many parameters as to make the system unusable, it however does involve the use and control of multiple drug infusions and inputs. Neither of these patented inventions are in widespread use due to their general non-specific nature and usefulness and both lack a visual or textual physiological balance indicator. These inventions also do not allow for the ability to automatically control blood pressure to a user selected value or a tight range. A physiological balance mechanism or indicator allows the user to determine at a glance how well balanced the system is according to a user set target pressure and range. Neither of the prior inventions incorporates a means of determining the physiologic balance. Also neither device accounts for variable sensitivities to certain vasoactive medications within the time course of treatment. These publications and all other referenced patents are incorporated herein by reference in their entirety. Furthermore, where a definition or use of a term in a reference, which is an incorporated reference here, is inconsistent or contrary to the definition of that term provided herein the definition of the term provided herein applies and the definition of that term in the reference does not apply. Document US 2013 / 0 261 472 A1 discloses a system for controlling the blood circulation by monitoring the mean arterial pressure and the cardiac output. Document US 2011 / 0 133 935 A1 discloses a wireless patient monitoring system employing room units to collect and display information from a variety of room based sensors. The room units may relay collected data from the associated sensor units and to a central controller.

### SUMMARY

The problem underlying the present application is solved by a medical device having the features of claim 1.

An automated pressure control device that is useful will allow the user to determine a target pressure and range as well as incorporate either a textual or visual physiologic balance indicator that will assist the device and user in maintaining its preset target pressure within its user defined range. The physiologic balance indicator interfaces with the user by providing advisories, cautions and warnings on how well the device is able to maintain its set target and range. User input by way of outside interventions such as increased fluids, decreased anesthetics etc. will almost always be required at some point to maintain a preset target pressure and range. By combining the technologies behind auto-pilots in aircraft with interactive 'smart' intravenous pumps, arterial wave form monitoring, and a combination of vasodilator and vasoconstrictive intravenous drugs, a useful device will be able to automatically control arterial systolic blood pressure or mean arterial blood pressure to a user defined target value and continue to maintain that pressure within a user set range. The device can automatically control the arterial systolic pressure and maintain it within a user set range far more accurately and with infinitely more vigilance than any human can do so by altering the drip rate of vasoactive drugs through a set of infusion pumps. Vasoactive drugs are rapidly metabolized and they require extreme vigilance to maintain a pressure within a predetermined range. Notwithstanding this but depending on the circumstances a vasodilator may be needed followed rapidly by a vasoconstrictor within minutes of each other as patient's pressures can vary rapidly and without warning. Physiological systems are very dynamic and not well characterized. Anesthesiologists witness different sensitivity of patients to the same medication and changes in the same patient's sensitivity to any drug in time (for example, tachyphylaxis, etc). It is desirable to use a controller or automated device that can rapidly adapt to the changing cardiovascular parameters of any particular patient or situation. The controller in the present invention rapidly updates its parameters based on the latency and response to current doses as well as test dosages in relation to the cardiovascular system and both vasoactive substances.

Still further advantages will become apparent from a study of the following description and the accompanying drawings.

### BRIEF DESCRIPTION OF THE DRAWINGS

FIG. 1 is a drawing of the invention with vasoactive drug filled bags attached to a patient and close-up view of a connector to central venous infusion line.
FIG. 2 shows a profile of a dual lumen catheter.
FIG. 3 is an overall view of a central venous catheter setup.
FIG. 4 shows the device interface of the device.

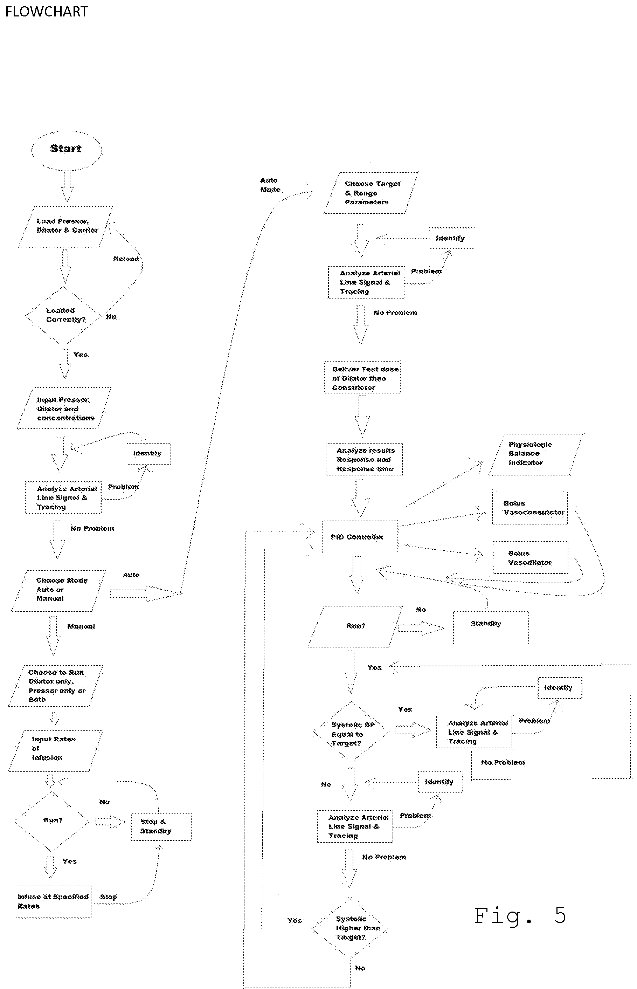

FIG. 5 is a flow chart illustrating operational steps of the present invention.

### DETAILED DESCRIPTION

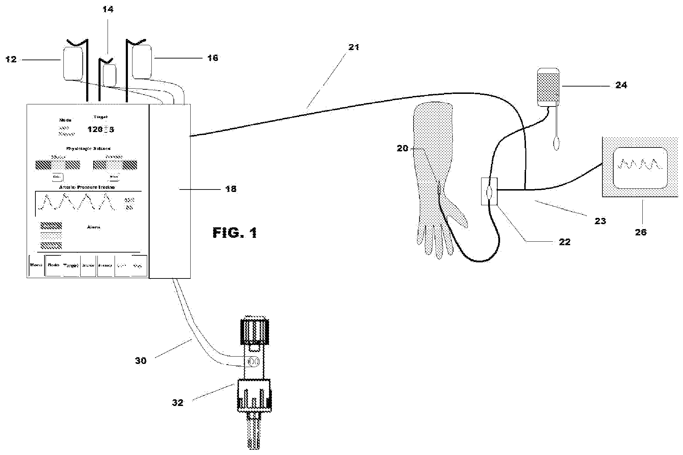

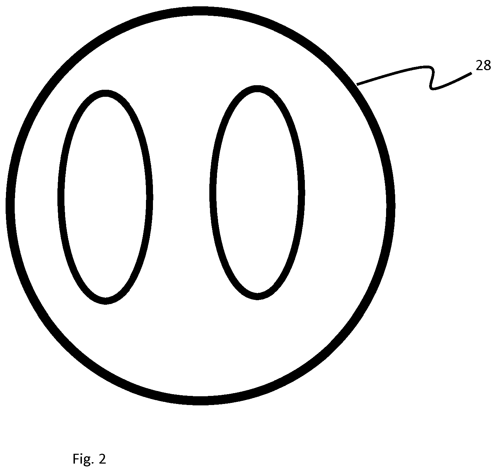

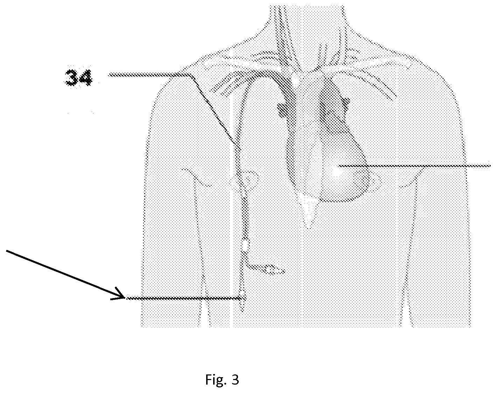

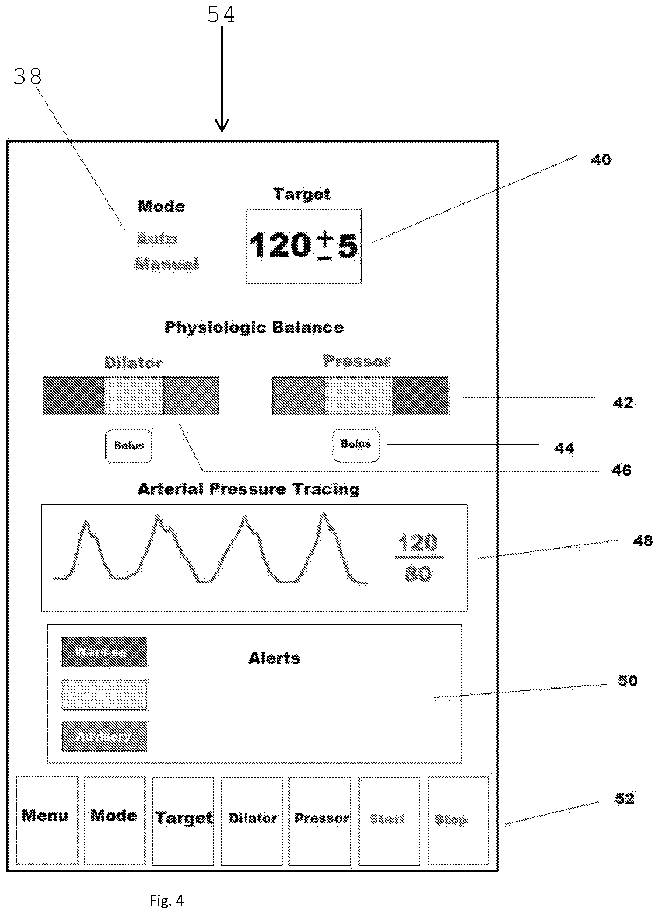

FIG. 1 is an overview of the device 10 showing vasodilator solution bag 12, carrier solution bag 14, vasoconstrictor solution bag 16 each with its own access tubing entering the intravenous line housing 18 on the right hand side of the device 10 that houses three separate IV pump channels. Each line will have a means for a safety lock device or alternatively a single standard lock for the coalesced lines exiting the bottom on the IV infusion compartment. Out of the bottom of the IV infusion compartment a single line emerges that is actually three tubing channels in one line. This multi lumen infusion line 30 then will go to the connector port 32 that will connect to a central venous access line 34 for the patient. Entering the backside of the device is a pressure transducer cable 21 that will interface with the existing transducer to bring the arterial line 20 pressure signal into the device 10. FIG. 1 shows an overview of a standard invasive radial arterial monitoring setup, with A-line 20, transducer 22, external pressure monitor 26, and pressure bag 24. A 'Y' transducer cable 23 will interface the transducer signal to both the external monitor 26 and the device 10 via cable 21. Alternatively a 'Y' arterial line will connect to a separate transducer that will interface with the device 10. The device 10 further comprises a computer with microprocessor and ram that has a full color display to interface with a physician or care giver. FIG. 2 shows a close up view of the multi lumen infusion line 30 and a cross-section view of a multi lumen line 28, showing that there are three lumens inside of the multi lumen infusion line 30. The multi lumen infusion line 30 leads to the connector port 32 which has a flow through design. One embodiment of this connector port 32 will have an anti-reflux valve at the top of the connector port 32 to prevent vasoactive solution moving up a piggybacked IV line if the connector port 32 has a flow through design. Another embodiment of this connector port 32 is just a sealed top and open infuser bottom. The connector port 32 is connected to the central venous line 34 that is very close to the patient and eliminates the need for large volumes of solution to be infused in to the patient or to clear lines or the need to flush the central venous line 34 between solution changes. FIG. 3 shows a standard central line 34 infusion setup on a patient. The preferred method of use is through a central line 34, but the device 10 may be used with a peripheral access line, however the response time of the patient will be slower due to blood flow considerations. The connector port 32 will connect to the central line 34 as shown by arrow 36. FIG. 4 shows one embodiment of the device 10 touch screen device interface 54 with user selected target and range window 40 in the upper right corner, mode annunciation 38 in the upper left, the physiologic balance indicator in the upper mid portion of the device face with the dilator balance 46 on the left, and the pressor balance 42 on the right. The pressor bolus button 44 is touched by the physician to dose the patient with a vasoconstrictor, the dilator bolus button is used by the physician to deliver a dose of vasodilator. In other words, this is a manual override and can be used while the device is in manual or auto mode. If things are happening quickly for example, say the surgeon just lifted the heart up and the blood pressure plummeted and the anesthesiologist needed a quick bolus the user could push one of the buttons and the device would deliver a quick bolus that the user has preselected a dosage for. In the lower mid portion of the device face is an arterial pressure tracing and arterial pressure reading 48. The Warning, Caution, Advisory and Alerts section 50 is just below the arterial pressure tracing and arterial pressure reading 48 area. Alerts section 50 signals warnings in red, cautions in yellow and advisories in grey and presents them in the alerts section 50. At the bottom of the touch screen interface is the menu area 52. Each menu button brings up a separate menu to address that specific menu item. For instance the mode button initiates a separate menu that has the user select a mode for the device to operate in, manual vs. auto mode and any specific menu items that m-e needed to operate the device in that mode. Along the bottom of the device interface 54 are touch screen buttons: "Menu" brings up a sub menu screen that allows the user to select the dosages for bolus buttons, and another sub menu to determine whether or not a baseline infusion of either dilator or constrictor is desired in auto mode and what that infusion rate would be. "Mode" button brings up a submenu that tells the user the current mode the device is in as well as a submenu that allows for various infusions and rates in the Manual mode. '"Target" brings up a submenu that asks for the range input and target pressure input. "Dilator" brings up a submenu that asks for the name of the specific vasodilator used and its concentration. It also gives a current readout of the total dilator used thus far in the case and the ability to reset the value. "'Pressor" brings up a submenu that asks for the pressor drug name and the concentration in the vasoconstrictor solution bag 16, as well as a readout of the current total dosage given and the ability to reset the value. "Start" brings up a submenu that asks if the user wishes to start the infusion in either Auto or Manual mode. "'Stop" brings up a submenu that asks the user if they wish to confirm stopping all infusions.

### REFERENCE NUMERALS

12 vasodilator solution bag
14 carrier solution bag
16 vasoconstrictor solution bag
18 intravenous line housing
20 Arterial Line
21 Transducer Cable
22 Arterial Pressure Transducer
23 Transducer Cable 'Y' Split
24 Arterial Line Pressure Bag
26 External Monitor
28 Cross Section of multi lumen infusion line
30 multi lumen infusion line
32 connector port
34 an example of a central venous line
36 connector port 32 connecting to central venous line 34
38 mode annunciation window
40 target and range window
42 pressor balance
44 Bolus Activation Button
46 dilator balance
48 arterial pressure tracing and arterial pressure reading
50 alert section
52 menu area
54 Device Interface

### OPERATION

In operation the device will be powered on and specialized vasoactive line tubing will be spiked into the vasoconstrictor solution bag 16 and vasodilator solution bag 12 as well as the carrier solution 14. Each solution line has a channel as in standard intravenous pumps. The vasoconstrictor line may he coded and colored red, the vasodilator line may be coded and colored blue to minimize errors. The device undergoes a standard intravenous line check Vasoactive solutions will be identified and input by the user including their respective concentrations. The device is capable of two modes, manual and auto. In manual mode the device will have the means to infuse either the dilator or constrictor or both at user specified rates. In auto mode the device will require an incoming arterial transducer line 21 with means to enable the device to read the arterial pressure signal, alternatively this will come from a 'Y' arterial line and separate transducer and line that will interface with the device. From this transducer line 21 the arterial waveform and derived systolic pressure will be displayed on the arterial pressure tracing and arterial pressure reading 48. If the waveform is too damped or unusable the device will go into standby mode until the tracing is corrected, and activate the alert window giving an alert section 50. In auto mode the user inputs a target systolic pressure with a range above and below the target value 40. The device will give a small test dose of each vasoactive solution in order to determine appropriate controller parameters. For example in the auto mode, an algorithm, PID controller, or similar controller analyzes the patient response to the drug and calculates a rate for drug infusion based on the user inputs for drug concentration. When the device is ready the user activates the auto mode and the device will maintain the user selected target pressure. The degree to which the device is 'fighting' to maintain target pressure will be displayed on the balance indicator 42, 46 along with alert section 50. Alerts are in the form of recommendations to the user in order to assist the device in maintaining the target value. In an alternative embodiment the device operates without the use of invasive blood pressure readings via a blood pressure cuff or one of the new continuous noninvasive blood pressure measuring devices.

The ability of the device to use any available vasopressors or vasodilators, such as nitroglycerin, nitroprusside, nicardipine, epinephrine, norepinephrine, phenylephrine or newly developed medications etc. are alternative embodiments.

In an alternative embodiment the device calculates adding an inotrope to the vasopressor bag to provide inotropic support as well as vasoconstriction.

In all of the embodiments the blood pressure measurements can be recorded locally on the device with removable memory or accessible via conventional USB or other connectors. Additionally, each embodiment can utilize WiFi, BlueTooth@, or other wireless communication to send patient data to a remote server for recording or remote analysis.

## Claims

1. A medical device configured to automatically control an arterial systolic blood pressure of a patient, comprising:
a controller configured to control the arterial systolic blood pressure of the patient,
a pump configured to deliver drugs to the patient via a multi-lumen infusion line (30),
at least one vasodilator connected to the controller and to the pump,
at least one vasoconstrictor connected to the controller and to the pump,
a pressure monitor (26) configured to monitor a blood pressure of the patient and connected to the controller,
a transducer (22) connected to a patient, and
a transducer cable (21) configured to interface the transducer (22) to both the pressure monitor (26) and the controller,
wherein the controller has a user interface (54) that has a touch screen having buttons (44) for a user to input data,
**characterized in that**
the input data include a user defined target arterial systolic blood pressure, and
when a blood pressure of a patient deviates from the user defined target arterial systolic blood pressure, the controller directs the pump to deliver a bolus of the at least one vasodilator or the at least one vasoconstrictor to maintain a measured systolic blood pressure at the user defined target arterial systolic blood pressure.

2. The medical device of claim 1, wherein when a blood pressure of a patient deviates from the target arterial systolic blood pressure or from the target mean arterial pressure, the controller alerts a user with an audio or visual alarm.

3. The medical device according to one of the claims 1 or 2, wherein the controller is configured to test the sensitivity of the patient to both the vasoconstrictor and the vasodilator and to continually update the change in sensitivity to those drugs while infusions are taking place.

4. The medical device of one of the claims 1 to 3, wherein the patient blood pressure is recorded on the device or at a remote location.

5. The medical device of claim 1, wherein the touch screen displays a patient blood pressure and the touch screen has a green, yellow, and red indicator that represents how a patient's current blood pressure is relative to the target blood pressure and the current dosages of the at least one vasodilator and the at least one vasoconstrictor.

6. The medical device of one of the claims 1 to 5, comprising a physiological balance indicator (42, 46) configured to display a patient's physiologic arterial pressure balance with regard to said device by textual, graphic, audio means, or a combination of textual, graphic, and audio means, and to assist the user in maintaining its preset target arterial systolic or mean pressure.

7. The medical device of claim 6, wherein the physiological balance indicator (42, 46) indicates separately a dilator balance (46) and a pressor balance (42).

8. The medical device according to one of the claims 6 or 7, wherein the physiological balance indicator (42, 46) is configured to interface with the user by providing advisories, cautions, and warnings on how well the device is able to maintain its target arterial systolic or mean pressure.

9. The medical device according to one of the claims 5 to 8, further comprising:
a carrier solution;
means for infusing the vasodilator, the vasoconstrictor, and the carrier solution into a patient
i. at a user defined rate,
ii. at said device determined rate; and
a memory with means to store information regarding various vasoactive solutions; whereby said device is configured to automatically regulate arterial systolic blood pressure to a user determined value with guidance from said device's physiologic balance indicator (42, 46).

## Patentansprüche

1. Medizinische Vorrichtung, die dazu konfiguriert ist, einen arteriellen systolischen Blutdruck eines Patienten automatisch zu steuern, umfassend:
eine Steuerung, die dazu konfiguriert ist, den arteriellen systolischen Blutdruck des Patienten zu steuern,
eine Pumpe, die dazu konfiguriert ist, dem Patienten Medikamente über eine Mehrlumen-Infusionsleitung (30) zu verabreichen,
mindestens einen Vasodilatator, der mit der Steuerung und mit der Pumpe verbunden ist,
mindestens einen Vasokonstriktor, der mit der Steuerung und mit der Pumpe verbunden ist,
eine Drucküberwachungseinrichtung (26), die dazu konfiguriert ist, einen Blutdruck des Patienten zu überwachen, und die mit der Steuerung verbunden ist,
einen Wandler (22), der mit einem Patienten verbunden ist, und
ein Wandlerkabel (21), das dazu konfiguriert ist, eine Schnittstelle für den Wandler (22) zu sowohl der Drucküberwachungseinrichtung (26) als auch der Steuerung herzustellen,
wobei die Steuerung eine Benutzerschnittstelle (54) aufweist, die einen Touchscreen mit Schaltflächen (44) aufweist, damit ein Benutzer Daten eingeben kann,
**dadurch gekennzeichnet, dass**
die eingegebenen Daten einen benutzerdefinierten Sollwert für den arteriellen systolischen Blutdruck beinhalten und
die Steuerung, wenn ein Blutdruck eines Patienten von dem benutzerdefinierten Sollwert für den arteriellen systolischen Blutdruck abweicht, die Pumpe anweist, eine Injektion des mindestens einen Vasodilatators oder des mindestens einem Vasokonstriktors abzugeben, um einen gemessenen systolischen Blutdruck auf dem benutzerdefinierten Sollwert für den arteriellen systolischen Blutdruck aufrechtzuerhalten.

2. Medizinische Vorrichtung nach Anspruch 1, wobei die Steuerung, wenn ein Blutdruck eines Patienten von dem Sollwert für den arteriellen systolischen Blutdruck oder von dem Sollwert für den mittleren arteriellen Druck abweicht, einen Benutzer mit einem akustischen oder optischen Alarm warnt.

3. Medizinische Vorrichtung nach einem der Ansprüche 1 oder 2, wobei die Steuerung dazu konfiguriert ist, die Empfindlichkeit des Patienten gegenüber sowohl dem Vasokonstriktor als auch dem Vasodilatator zu testen und die Änderung der Empfindlichkeit gegenüber diesen Arzneimitteln kontinuierlich zu aktualisieren, während Infusionen erfolgen.

4. Medizinische Vorrichtung nach einem der Ansprüche 1 bis 3, wobei der Blutdruck des Patienten in der Vorrichtung oder an einem entfernten Ort aufgezeichnet wird.

5. Medizinische Vorrichtung nach Anspruch 1, wobei der Touchscreen den Blutdruck eines Patienten anzeigt und der Touchscreen einen grünen, gelben und roten Indikator aufweist, der das Verhältnis des aktuellen Blutdrucks eines Patienten zu dem Sollwert für den Blutdruck und die aktuelle Dosierung des mindestens einen Vasodilatators und des mindestens einen Vasokonstriktors darstellt.

6. Medizinische Vorrichtung nach einem der Ansprüche 1 bis 5, umfassend einen Indikator (42, 46) für das physiologische Gleichgewicht, der dazu konfiguriert ist, das physiologische arterielle Druckgleichgewicht eines Patienten im Hinblick auf die Vorrichtung durch textliche, grafische, akustische Mittel oder eine Kombination aus textlichen, grafischen und akustischen Mitteln anzuzeigen und den Benutzer beim Aufrechterhalten seines voreingestellten Sollwerts für den arteriellen systolischen oder mittleren Druck zu unterstützen.

7. Medizinische Vorrichtung nach Anspruch 6, wobei der Indikator (42, 46) für das physiologische Gleichgewicht separat ein Dilatatorgleichgewicht (46) und ein Pressorgleichgewicht (42) angibt.

8. Medizinische Vorrichtung nach einem der Ansprüche 6 oder 7, wobei der Indikator (42, 46) für das physiologische Gleichgewicht dazu konfiguriert ist, eine Schnittstelle mit dem Benutzer herzustellen, indem er Hinweise, Vorsichtsmaßnahmen und Warnungen darüber bereitstellt, wie gut die Vorrichtung in der Lage ist, seinen Sollwert für den arteriellen systolischen oder mittleren Druck aufrechtzuerhalten.

9. Medizinische Vorrichtung nach einem der Ansprüche 5 bis 8, ferner umfassend:
eine Trägerlösung;
Mittel zum Infundieren des Vasodilatators, des Vasokonstriktors und der Trägerlösung in einen Patienten
i. mit einer benutzerdefinierten Rate,
ii. mit der durch die Vorrichtung bestimmten Rate; und
einen Speicher mit Mitteln zum Speichern von Informationen über verschiedene vasoaktive Lösungen; wobei die Vorrichtung dazu konfiguriert ist, den arteriellen systolischen Blutdruck unter Anleitung von dem Indikator (42, 46) für das physiologische Gleichgewicht der Vorrichtung automatisch auf einen durch den Benutzer bestimmten Wert zu regeln.

## Revendications

1. Dispositif médical configuré pour commander automatiquement une pression artérielle systolique d'un patient, comprenant :
un dispositif de commande configuré pour commander la pression artérielle systolique du patient,
une pompe configurée pour administrer des médicaments au patient via une ligne de perfusion à plusieurs lumières (30),
au moins un vasodilatateur connecté au dispositif de commande et à la pompe,
au moins un vasoconstricteur connecté au dispositif de commande et à la pompe,
un dispositif de surveillance de la pression (26) configuré pour surveiller une pression artérielle du patient et connecté au dispositif de commande,
un transducteur (22) connecté à un patient, et
un câble de transducteur (21) configuré pour interfacer le transducteur (22) à la fois au dispositif de surveillance de pression (26) et au dispositif de commande,
dans lequel le dispositif de commande présente une interface utilisateur (54) qui présente un écran tactile présentant des boutons (44) permettant à l'utilisateur de saisir des données, **caractérisé en ce que**
les données saisies comportent une pression artérielle systolique cible définie par l'utilisateur, et
lorsqu'une pression artérielle d'un patient s'écarte de la pression artérielle systolique cible définie par l'utilisateur, le dispositif de commande ordonne à la pompe de délivrer un bolus de l'au moins un vasodilatateur ou de l'au moins un vasoconstricteur pour maintenir une pression artérielle systolique mesurée au niveau de la pression artérielle systolique cible définie par l'utilisateur.

2. Dispositif médical selon la revendication 1, dans lequel, lorsqu'une pression artérielle d'un patient s'écarte de la pression artérielle systolique cible ou de la pression artérielle moyenne cible, le dispositif de commande alerte un utilisateur par une alarme sonore ou visuelle.

3. Dispositif médical selon l'une des revendications 1 ou 2, dans lequel le dispositif de commande est configuré pour tester la sensibilité du patient au vasoconstricteur et au vasodilatateur et pour mettre continuellement à jour le changement de sensibilité à ces médicaments pendant que les perfusions se déroulent.

4. Dispositif médical selon l'une des revendications 1 à 3, dans lequel la pression artérielle du patient est enregistrée sur le dispositif ou à un emplacement distant.

5. Dispositif médical selon la revendication 1, dans lequel l'écran tactile affiche la pression artérielle d'un patient et l'écran tactile présente un indicateur vert, jaune, et rouge qui représente la manière dont la pression artérielle actuelle d'un patient se situe par rapport à la pression artérielle cible et aux doses actuelles de l'au moins un vasodilatateur et de l'au moins un vasoconstricteur.

6. Dispositif médical selon l'une des revendications 1 à 5, comprenant un indicateur d'équilibre physiologique (42, 46) configuré pour afficher l'équilibre de pression artérielle physiologique d'un patient par rapport audit dispositif par des moyens textuels, graphiques, audio, ou une combinaison de moyens textuels, graphiques, et audio, et pour aider l'utilisateur à maintenir sa pression artérielle systolique ou moyenne cible prédéfinie.

7. Dispositif médical selon la revendication 6, dans lequel l'indicateur d'équilibre physiologique (42, 46) indique séparément un équilibre de dilatation (46) et un équilibre de pression (42).

8. Dispositif médical selon l'une des revendications 6 ou 7, dans lequel l'indicateur d'équilibre physiologique (42, 46) est configuré pour interagir avec l'utilisateur en fournissant des conseils, des mises en garde, et des avertissements sur la capacité du dispositif à maintenir sa pression artérielle systolique ou moyenne cible.

9. Dispositif médical selon l'une des revendications 5 à 8, comprenant également :
une solution porteuse ;
des moyens de perfusion du vasodilatateur, du vasoconstricteur, et de la solution porteuse chez un patient
i. à un débit défini par l'utilisateur,
ii. à un débit déterminé par ledit dispositif ; et
une mémoire possédant des moyens de stockage d'informations concernant diverses solutions vasoactives ; moyennant quoi ledit dispositif est configuré pour réguler automatiquement la pression artérielle systolique à une valeur déterminée par l'utilisateur avec des indications de l'indicateur d'équilibre physiologique dudit dispositif (42, 46).
